# EUROPÄISCHE PATENTSCHRIFT

(11) **EP 0 999 780 B1**
(45) Veröffentlichungstag und Bekanntmachung des Hinweises auf die Patenterteilung: **25.02.2004**
(21) Anmeldenummer: 98943818.9
(22) Anmeldetag: 31.07.1998
(51) Int. Cl.: A61B 1/002

(54) **ENDOSKOP MIT FIXIERUNG FÜR BAUELEMENTE DES OPTISCHEN SYSTEMS SOWIE VERFAHREN ZUM MONTIEREN DER BAUELEMENTE**
ENDOSCOPE WITH FIXING UNIT FOR COMPONENTS OF THE OPTICAL SYSTEM AND METHOD FOR ASSEMBLING SAID COMPONENTS
ENDOSCOPE COMPORTANT UN SYSTEME DE FIXATION POUR COMPOSANTS DU SYSTEME OPTIQUE, AINSI QUE PROCEDE DE MONTAGE DES COMPOSANTS

(30) Priorität: 31.07.1997 DE 19732991
(43) Veröffentlichungstag der Anmeldung: 17.05.2000
(73) Patentinhaber: Karl Storz GmbH & Co. KG, 78532 Tuttlingen (DE)
(72) Erfinder: KEHR, Ulrich, D-73760 Ostfildern (DE); RUDISCHHAUSER, Jürgen, D-78532 Tuttlingen (DE)
(74) Vertreter: Weller, Wolfgang, Dr.
(86) Internationale Anmeldenummer: PCT/EP1998/004813
(87) Internationale Veröffentlichungsnummer: WO 1999/005959

(56) Entgegenhaltungen:
- EP-A- 0 260 855
- EP-A- 0 292 602
- WO-A-90/04350
- DE-U- 9 318 440

## Beschreibung

Die vorliegende Erfindung betrifft ein Endoskop mit einem Rohrschaft, in dessen Innerem zumindest ein Bauelement, insbesondere Linsen, Blenden, Filter, eines optischen Systems angeordnet ist, wobei zwischen der Außenseite des Bauelementes und der Innenseite des Rohrschafts zumindest ein das Bauelement zumindest teilweise umgebendes Stützelement vorgesehen ist.

Ein derartiges Endoskop ist aus der US 4,148,550 bekannt.

Endoskope werden allgemein in der Chirurgie dazu verwendet, Körperhohlräume und Hohlorgane zu inspizieren. Endoskope weisen einen langerstreckten Rohrschaft auf, in dem Bauelemente eines optischen Beobachtungssystems angeordnet sind. Häufig sind im Inneren des Endoskopschafts weitere Vorrichtungen, meist eine Lichtzuführvorrichtung, und ggf. Kanäle für Instrumente, Spülflüssigkeit oder dgl. vorgesehen. Die Lichtzuführung erfolgt meist über lichtleitende Glasfasern.

In diesem Fall ist das optische System in einem separaten Innenrohr im Endoskopschaft aufgenommen.

Endoskope können starr oder flexibel ausgebildet sein, wobei der Endoskopschaft entsprechend aus Metall oder einem flexiblen Kunststoffmaterial hergestellt ist.

Die in Endoskopen vorgesehenen optischen Systeme sind aus entlang der Rohrschaftachse angeordneten Bauelementen wie Linsen, insbesondere Stablinsen der sogenannten HOPKINS-Optik, Blenden, Filtern und dgl. aufgebaut und haben die Aufgabe, eine klare Abbildung eines möglichst großen Blickfelds mit großer Auflösung und hohem Kontrast wiederzugeben. Die wichtigste Voraussetzung hierfür ist die präzise Anordnung der Bauelemente des optischen Systems im Rohrschaft des Endoskops. Die relativen Positionen der Bauelemente des optischen Systems sind nicht variabel, sondern genau definiert, denn jede Verschiebung der Bauelemente relativ zueinander führt zu einer verminderten Bildschärfe und Auflösung.

Demnach müssen die Bauelemente präzise und dort fest verbleibend im Rohrschaft des Endoskops fixiert werden.

Üblicherweise werden die Bauelemente eines optischen Systems in den Rohrschaft des Endoskops eingeschoben, untereinander durch Distanzstücke auf Abstand gehalten und durch endseitige Abschlußelemente fixiert. Diese Art der Fixierung ist jedoch unzureichend, da aufgrund von Toleranzen ein radiales Hin- und Herbewegen erfolgen kann. Zwischen der Außenseite der Bauelemente und Innenseite des Rohrschafts ist ein dünner Luftspalt vorhanden, bzw. die Bauelemente liegen über ihre Außenseite an der Innenseite des Rohrschafts an.

Außerdem können vor allem die aus Glasmaterial hergestellten Linsen des optischen Systems bei der Handhabung des Endoskops, bspw. wenn das Endoskop zu fest auf eine harte Unterlage gelegt wird oder herabfällt, zerbrechen, da aufgrund des nur dünnen Luftspaltes zwischen Bauelement und Rohrschaft schon bei geringem Verbiegen des Rohrschafts Kräfte bzw. Momente auf die Bauelemente einwirken, die bei Linsen zu Brüchen führen können.

Diesem Problem wurde bspw. bei den Stablinsen durch eine Knochenform der Linse begegnet, also einer Form, bei der die Enden der Stablinse Verdickungen, sogenannte Randzylinder, aufweisen. Die formschlüssige Verbindung zu dem Rohrschaft erfolgt dann nur im Bereich der Randzylinder, der übrige Umfang der Stablinse ist durch einen Spalt von dem Rohrschaft getrennt.

Bei Reinigung treten Erschütterungen auf (Spülmaschine, Ultraschall), die dazu führen, daß die Linsen aufgrund Ihres Radialspiels gegen das Innenrohr schlagen. Außerdem treten Stöße zwischen Linse und Abstandselement auf (Abtrieb, Verschmutzung).

Eine Lösung, wie die Linsen eines optischen Systems in einem Endoskop untereinander verbunden werden können, ist in der eingangs genannten US 4,148,550 beschrieben.

Das dort beschriebene Endoskop weist ein äußeres Schutzrohr, an dessen Innenseite eine Schicht mit lichtübertragenden Fasern vorgesehen ist, sowie ein Innenrohr auf, in dem eine Anzahl von Stablinsen, die ein optisches System bilden, axial hintereinanderliegend angeordnet sind.

Für die Funktion des optischen Systems ist es, wie bereits erwähnt, notwendig, die Stablinsen in einem definierten axialen Abstand zueinander in dem inneren Rohr des Endoskops anzuordnen. Hierzu werden im Stand der Technik allgemein Abstandshalter in Form von meist aus Metall bestehenden Abstandsröhrchen verwendet.

In der US 4,148,550 wird die Verbindung zwischen den Stablinsenenden durch schlauchförmige Elemente hergestellt, die die Linsenenden zumindest zweier benachbarter Linsen umschließen.

Diese Elemente bestehen aus einem flexiblen schlauchförmigen Material und diese weisen sich längserstreckende Schlitze auf, die zur Einführung eines Werkzeuges und zum Durchführen von reinigenden Gasen oder Flüssigkeiten für die Linsen vorgesehen sind. Außer einem Zusammenhalten der hintereinander angeordneten Stablinsen dienen die Elemente auch dazu, eine Biegung des flexiblen Endoskops zu erlauben, ohne daß die Stablinsen dabei zerbrechen. Diese Elemente selbst können schon für eine Abstützung gegenüber der Innenwand des Rohrschafts dienen, es können auch noch zusätzliche ringförmige Stützelemente vorgesehen sein.

Die Befestigung bzw. Fixierung der durch die Stützelemente verbundenen Stablinsen im Innenrohr in dem Endoskop erfolgt durch Einklemmen, und zwar z.B. dadurch, daß dem das Stablinsensystem umschließende innere zunächst zylindrische Rohr ein leicht ovaler oder dreieckiger Querschnitt verliehen wird, so daß die im Querschnitt runden Stablinsen eingeklemmt festsitzen. Es wird weiter vorgeschlagen, geeignete Klebstoffe einzusetzen.

Die in dieser US-Patentschrift vorgesehenen Elemente sorgen für eine Verbindung der Stablinsen untereinander, und für eine Abstützung mit den sie umgebenden Teilen des Endoskops. Die axial unverrückbare Verbindung muß gesondert bewerkstelligt werden, da sonst die Linsenanordnung als Ganzes sich im Inneren des Endoskops axial hin- und herbewegen kann, was wegen der Beeinträchtigung der optischen Qualität sowie wegen der Gefahr eines Bruchs von Linsenteilen unerwünscht ist.

Aus der DE 39 12 720 C2 ist ein Endoskop bekannt, dessen Rohrschaft aus einem durchsichtigen und optisch klaren Kunststoffmaterial besteht, in den die optischen Elemente des Objektivs und des Linsensystems eingesetzt sind. Dieser Hohlzylinder dient als Lichtleiter um das Beleuchtungslicht vom proximalen zum distalen Ende des Endoskops zu leiten.

Um die in dem Hohlzylinder eingesetzten Linsen vor Lichteintritt aus dem Lichtleitsystem abzuschirmen, ist zwischen dem Hohlzylinder und dem Objektiv bzw. dem Linsensystem ein lichtabsorbierendes und/oder ein reflektierendes Element vorgesehen. Dieses Element kann als ein Kunststoff-Schrumpfschlauch ausgebildet sein. In diesen Kunststoff-Schrumpfschlauch werden zunächst die Linsen des Linsensystems und das Objektiv eingesetzt und in der richtigen Stellung positioniert, anschließend wird der Schrumpfschlauch durch Anwendung von Wärme geschrumpft, so daß er die Linsen hält. Anschließend wird dieser Zusammenbau in den Rohrschaft eingeschoben. Der Kunststoff-Schrumpfschlauch dient somit dazu, die Linsen unter einander in bestimmter Ausrichtung zu halten und außerdem als lichtabsorbierendes oder lichtreflektierendes Element.

Aus der DE 34 31 631 C2 ist eine Linsenhalterung zur Radialjustierung von Stablinsen in einem Optikrohr im Bereich der Linsenenden bekannt, bei der ringförmige Halterungsmittel vorgesehen sind, die die Linsen im Radialabstand zur Rohrinnenfläche halten.

Vor diesem Hintergrnd ist es Aufgabe der vorliegenden Erfindung, ein Endoskop der eingangs genannten Art zu schaffen, bei dem die Bauelemente des optischen Systems möglichst einfach und dennoch sicher im Inneren des Rohrschafts fixiert und zudem gegenüber äußeren Krafteinwirkungen bzw. Momenteneinwirkung geschützt werden können.

Die Erfindung ist im Anspruchssatz definiert.

Die Aufgabe wird bei einem Endoskop dadurch gelöst, daß das Stützelement aus einem schrumpffähigen Material hergestellt ist und daß das Bauelement über das im Rohrschaft geschrumpfte Stützelement an der Innenseite des Rohrschafts fixiert ist.

Unter einem schrumpffähigen Material im Sinne der vorliegenden Erfindung wird einerseits ein kaltgerecktes, thermoplastisches Kunststoffmaterial verstanden, das sich bei Wärmebehandlung wieder auf seinen Urzustand zusammenzieht. Der Schrumpfvorgang beruht auf dem Bestreben der Kunststoffmoleküle, zu ihrer ursprünglichen spannungsfreien Anordnung zurückzukehren. Man nennt dies auch Rückerinnerungsvermögen oder elastisches Formgedächtnis. Beispiele für schrumpffähige Materialien sind PETP, PE oder PVC.

Unter einem schrumpffähigen Material wird andererseits eine Metallegierung oder ein Kunststoffmaterial mit einem Memory-Effekt verstanden, das zumindest zwei Zustände aufweist, wobei ein Zustand über eine Materialschrumpfung erreicht wird. Bei den für die Erfindung in Frage kommenden Materialien muß der Memory-Effekt nach dem Schrumpfen aufgehoben sein.

Diese Art der Fixierung mit schrumpffähigen Stützelementen ist denkbar einfach, da das Bauelement zunächst zusammen mit dem Stützelement im ungeschrumpften, gereckten Zustand gerade passend in den Rohrschaft eingebracht und zutreffend positioniert werden kann. Durch die Wärmebehandlung schrumpft das Material, zieht sich also zusammen und wird in den Spalt zwischen Außenseite des Bauelements und Innenseite des Rohrschafts gepreßt. Dabei können, je nach Ausgestaltung der Stützelemente, Materialien eingesetzt werden, die in Richtung der Rohrschaftachse schrumpfen und/oder auch solche die in Querrichtung schrumpfen. Entscheidend ist, daß das schrumpfende Material den Spalt zwischen Bauelement und Rohrschaft derart ausfüllt, daß das Bauelement über das geschrumpfte Stutzelement an der Innenseite des Rohrschaftes fixiert ist. Dabei paßt sich das erwärmte Stützelement sowohl an die äußere Form des Bauelements als auch an die innere Form des Rohrschafts unabhängig von deren individueller Ausgestaltung an. Nach Erkalten füllt das geschrumpfte Material über gewisse axiale und umfängliche Bereiche hinweg in radialer Richtung den Luftspalt zwischen Außenseite des Bauelements und Innenseite des Rohrschafts aus. Aufgrund der Tatsache, daß das Material beim Schrumpfen in diesem Spalt geschrumpft ist, sich also radial ausdehnen wollte, dies aber wegen des dünnen Spaltes begrenzt ist, übt das geschrumpfte Material einen radial beidseitig wirkenden Anpreßdruck aus, der für eine ausreichende Fixierung der axialen und radialen Relativlage von Bauelement und Rohrschaft untereinander sorgt. Es sind weder Klebstoffe noch sonstige Befestigungsmittel oder Klemmverbindungen notwendig, ebensowenig muß ein genaues Einpassen der Bauelemente in den Rohrschaft erfolgen, da hier das zunächst gereckte und danach erst in der geschrumpften Form vorliegende Stützelement die Fixierung sicherstellt.

Das Vorsehen eines geschrumpften Stützelements aus einem Kunststoffmaterial hat den weiteren erheblichen Vorteil, daß es zusätzlich einen Schutz der Bauelemente des optischen Systems gegen äußere Krafteinwirkungen darstellt, da es elastisch ist und Stöße daher dämpfen kann. So wird selbst bei einem Herabfallen des Endoskops die Gefahr eines Zerbrechens der Bauelemente des optischen Systems erheblich verringert.

Dabei können die Bauelemente des optischen Systems Linsen, Filter, Blenden, Objektivpatronen oder alle sonstigen denkbaren in Endoskopoptiken verwendeten Bauelemente sein. Als Linsen kommen Stablinsen oder herkömmliche Linsen in Betracht, wobei die Stablinsen als glatte Zylinder oder in Knochen-, Tonnenoder jeder weiteren möglichen Form vorgesehen sein können. Üblicherweise besteht das optische System aus mehreren axial hintereinander angeordneten Bauelementen, die vorteilhafterweise alle, ggf. jedoch auch nur zwei benachbarte, jeweils mit einem erfindungsgemäßen geschrumpften Stützelement untereinander verbunden und zugleich im Rohrschaft befestigt werden können.

Unter einem Rohrschaft im Sinne der Erfindung wird jedes rohrförmige Teil eines Endoskops verstanden, in dem das optische System angeordnet ist. Dies wird meist ein Innenrohr sein, das zusammen mit weiteren Rohren für den Lichtleiter, Spülkanal o.ä. von einem Außenrohr umschlossen ist. Wenn keine weiteren Kanäle im Endoskopinneren vorgesehen sind, kann der Rohrschaft jedoch auch der Außenschaft des Endoskops sein. Der Rohrschaft eines erfindungsgemäßen Endoskops muß nicht starr sein, sondern kann auch flexibel sein, denn das geschrumpfte Stützelement ist selbst flexibel und folgt daher eine Biegung des Rohrschafts, ohne daß die Fixierung des Bauelements an der Innenseite des Rohrschafts sich lockert.

Die Auswahl des Materials bzw. dessen Schrumpftemperatur, richtet sich auch nach der Art des Endoskopes und dessen Einsatzzweck. Endoskope im medizinischen Bereich werden bei ca. 140°C sterilisiert, so daß Materialien mit Erweichungspunkten von größer als 140°C eingesetzt werden. Sind Materialien mit Erweichungstemperaturen unter 140°C einzusetzen, kann eine Isolierung vorgesehen sein, die insbesondere beim sogenannten Blitzautoklavieren dafür sorgt, daß im Innern des Endoskopschaftes die Erweichungstemperatur nicht erreicht wird. Temperaturen bis 200°C ertragen die gängigen optischen Kitte, die beispielsweise dazu verwendet werden, den Rohrschaft distalseitig mit einem optischen Fenster zu verschließen. Werden Materialien eingesetzt, die erst bei noch höheren Temperaturen (>200° - 400°C) schrumpfen, wird dafür Sorge getragen, daß nur das schrumpffähige Material diesen hohen Temperaturen ausgsetzt wird.

In einer bevorzugten Ausgestaltung besteht das Stützelement aus einem Schlauch, der einen Großteil der Außenseite des Bauelements bedeckt.

Das Vorsehen eines solchen Schrumpfschlauchs hat den Vorteil, großflächig eine besonders feste und sichere Fixierung des Bauelements an dem Rohrschaft zu erzielen, da der Schrumpfschlauch das Bauelement sozusagen in sich aufnimmt, also umfänglich umschließt, und somit zusätzlich die Bauelemente allseitig gegen Krafteinwirkungen von außen schützt.

Es versteht sich, daß auch Abstandshalter, wie bspw. aus Metall oder Kunststoff bestehende Abstandsröhrchen, zur Sicherstellung der definierten Abstände zwischen den Bauelementen des optischen Systems vorgesehen werden können, die dann ebenfalls durch das erfindungsgemäße Stützelement fixiert werden. Derartige Abstandshalter können jedoch auch zwischen den über das Stützelement fixierten Bauelementen ohne weitere Fixierung angeordnet werden.

In einer weiteren bevorzugten Ausführungsform besteht das Stützelement aus zumindest einem Ring.

Ein einziger Ring wird vorteilhafterweise zur Fixierung einer in Knochenform vorliegenden Stablinse eingesetzt, die durch die in den Endbereichen der Stablinsen vorgesehenen Randzylinder zusätzlich in dem Rohrschaft kippfrei wird. Der Ring wird dann vorteilhafterweise im mittleren Bereich der knochenförmigen Stablinse angeordnet. Ringförmige Stützelemente sind ferner dann besonders vorteilhaft, wenn schmale Linsen oder andere schmale Bauelemente fixiert werden sollen. Der Ring umschließt und fixiert dann den ganzen Umfang eines solchen schmalen Bauelements.

In einer besonders bevorzugten Ausgestaltung besteht das Stützelement aus zwei Ringen, die an gegenüberliegenden Enden des Bauelements angeordnet sind.

Diese Maßnahme hat den Vorteil, daß durch zwei axial voneinander beabstandete Stützelemente ein Verkippen des Bauelements innerhalb des Rohrschafts sicher verhindert wird. Außerdem ist vorteilhaft, daß ringförmige Stützelemente beim Zusammenbau des Endoskops besonders einfach auf Stablinsen aufzuschieben sind. Es versteht sich, daß auch mehr als zwei Ringe als Stützelemente eingesetzt werden können, vor allem bei sich langerstreckenden Bauelementen.

In einer weiteren Ausgestaltung besteht das Stützelement aus zumindest einem Halbring.

Diese Maßnahme hat den Vorteil, auf materialsparende Art und Weise die erfindungsgemäße Fixierung des Bauelements mit dem Rohrschaft zu bewerkstelligen. Es versteht sich, daß auch mehrere Halbringe, die ggf. alternierend auf gegenüberliegenden Seiten des Bauelements angeordnet werden, vorgesehen sein können.

Dadurch, daß ein Bauelement nicht vollständig umfänglich vom Stützelement umgriffen ist, erlaubt den axialen Durchtritt von Gasen z.B. zum Druckausgleich oder den Durchtritt von Spülflüssigkeiten.

In einer weiteren bevorzugten Ausführung besteht das Stützelement aus zumindest zwei Streifen, die sich in Längsrichtung des Rohrschafts erstrecken.

Solche Streifen sichern die Befestigung über deren gesamte Länge, so daß auch sich axial langerstreckende Bauteile wie Stablinsen sicher fixiert werden können, auch wenn sie nicht in Knochenform vorliegen, ohne daß ein Verkippen innerhalb des Rohrschafts auftreten kann. Es versteht sich, daß auch mehr als zwei Streifen vorgesehen werden können, wobei die Anordnung von drei Streifen besonders vorteilhaft ist, wenn sie gleichmäßig um den Umfang des Bauelements verteilt werden. Ein derartiger erfindungsgemäßer Streifen muß sich selbstverständlich nicht über die gesamte Länge des Bauelements erstrecken, sondern kann auch wesentlich kürzer sein, bis hin zu sogenannten "Pads" aus schrumpffähigem Material. Solche Pads können beliebig auf der Außenseite des Bauelements verteilt werden.

Ein Druckausgleich findet selbstverständlich auch bei der Verwendung von Streifen statt.

In einer weiteren besonders bevorzugten Ausgestaltung besteht das Stützelement aus einem schraubenlinienförmig gewundenen Band.

Ein solches schraubenlinienförmig gewundenes Band schützt die Außenseite des Bauelements umfänglich und sorgt zusätzlich für die Möglichkeit eines Druckausgleichs zwischen den beiden axialen Enden des Bauelements. Bei Luftdruckdifferenzen zwischen den beiden Enden können nämlich Längskräfte auftreten, die sich möglicherweise auf die axiale Position der Bauelemente auswirken.

In einer weiteren bevorzugten Ausführung ist die Außenseite des Bauelements glatt.

Diese Maßnahme hat den Vorteil, daß bspw. schlauchförmige Stützelemente einfach auf das Bauelement aufgeschoben werden können.

In einer weiteren Ausgestaltung weist die Außenseite des Bauelements zumindest eine Nut auf, und das Stützelement ist im Bereich der Nut angeordnet.

Diese Maßnahme hat den Vorteil, daß eine besonders innige Verbindung der Außenseite des Bauelements mit dem geschrumpften Material des Stützelements erreicht wird. Bei der für das Schrumpfen des schrumpffähigen Materials notwendigen Wärmebehandlung verkürzt sich das Material unter axialer Dickenzunahme, wie bereits erläutert. Dabei wird die Nut von dem Stützelement aufgefüllt. Das in die Nut eintretende Material sperrt, nach Erkalten, eine axiale Relativverschiebung zwischen Stützelement und Bauelement.

In einer weiteren besonders bevorzugten Ausführung weist die Außenseite des Bauelements zumindest einen Steg auf, und das Stützelement ist im Bereich des Stegs angeordnet.

Das geschrumpfte Schrumpfelement umschließt den Steg. Es können auch mehrere Stege vorgesehen sein, zwischen denen das Stützelement angeordnet ist, wobei das Bauelement in diesem Fall zusätzlich über die Stege im Rohrschaft abgestützt wird. Es versteht sich, daß die Form des Stegs rechteckig, spitz oder rundlich sein kann, je nach den Gegebenheiten des jeweiligen Bauelements. Der in das Material hineinragende und von diesem umschlossene Steg sperrt mechanisch eine Relativbewegung zwischen Stützelement und Bauelement.

In einer weiteren bevorzugten Ausgestaltung ist die Innenseite des Rohrschafts glatt.

Diese Maßnahme hat den Vorteil, daß der Zusammenbau aus Stützelement und Bauelement einfach in den Rohrschaft eingeschoben werden kann.

In einer weiteren Ausführung weist die Innenseite des Rohrschafts zumindest eine Nut auf, und das Stützelement ist im Bereich der Nut angeordnet.

Auch bei einer an der Innenseite des Rohrschafts angeordneten Nut kann sich das schrumpffähige Kunststoffmaterial des Stützelements während der Wärmebehandlung in die Nut hinein ausdehnen, diese auffüllen und dann eine axiale Relativbewegung zwischen Rohrschaft und Stützelement sperren.

In einer Ausgestaltung weist die Wand des Rohrschafts zumindest eine nach außen gerichtete Ausbauchung in Form eines Kugelabschnitts auf, und das Stützelement ist im Bereich der Ausbauchung angeordnet.

Diese Maßnahme hat den erheblichen Vorteil, das Bauelement besonders gut vor Krafteinwirkungen von außen zu schützen, da gerade im Bereich der Auflagefläche der Rohrschaftwand, nämlich über eine oder mehrere Ausbauchungen, eine besonders dicke wulstartige Schicht des dämpfenden Materials zwischen dem Bauelement und der Rohrschaftwand vorhanden ist.

In einer weiteren Ausgestaltung weist die Innenseite des Rohrschafts zumindest einen Steg auf, und das Stützelement ist im Bereich des Stegs angeordnet.

In diesem Fall wird der Steg von dem Stützelement umschlossen und sperrt eine axiale Verschiebung des Stützelements relativ zur Innenseite des Rohrschafts.

In einer bevorzugten Ausgestaltung sind mehrere Bauelemente axial im Inneren des Rohrschafts längs der Rohrschaftachse angeordnet, das Stützelement erstreckt sich über die Bauelemente hinweg und diese sind so in einer axialen Anordnung zueinander fixiert.

Diese Ausgestaltung hat den erheblichen Vorteil, daß das Stützelement nicht nur die Fixierung der Bauelemente an der Innenseite des Rohrschafts gewährleistet, sondern daß die einzelnen axial hintereinander angeordneten Bauelemente des optischen Systems auch relativ zueinander fixiert werden. So kann nicht nur eine axiale Verschiebung der einzelnen Bauelemente in dem Rohrschaft verhindert werden, sondern der für ein einwandfreies Funktionieren des optischen Systems notwendige Abstand der einzelnen Bauelemente des Systems wird zusätzlich fixiert und gesichert. Auf diese Weise erfüllt das erfindungsgemäße Stützelement eine dreifache Funktion: Zum ersten werden die Bauelemente an der Innenseite des Rohrschafts fixiert, zum zweiten werden die Bauelemente untereinander verbunden und relativ zueinander fixiert, und zum dritten werden die Bauelemente über deren gesamte Länge vor äußeren Krafteinwirkungen geschützt, da diese durch das elastische Material gedämpft werden. Eine Bewegung der Bauelemente des optischen Systems im Rohrschaft, die die Optik beeinträchtigen, ist somit praktisch ausgeschlossen.

Die Aufgabe der Erfindung wird ferner durch ein Verfahren zum Montieren von Bauelementen, insbesondere Linsen, Blenden, Filtern eines optischen Systems im Inneren eines Rohrschafts eines Endoskops, gelöst, bei dem auf die Außenseite des Bauelements des Rohrschafts zumindest ein das Bauelement zumindest teilweise umgebendes Stützelement gebracht wird und dieser Zusammenbau in den Rohrschaft eingeführt wird, das dadurch gekennzeichnet ist, daß ein Stützelement aus einem schrumpffähigen Material aufgebracht wird und daß nach Einführen des Zusammenbaus in den Rohrschaft eine solche Wärmebehandlung durchgeführt wird, daß das Material das Stützelement schrumpft, wodurch der Zusammenbau an der Innenseite des Rohrschafts fixiert wird.

Dieses Verfahren zum Montieren von Bauelementen ist besonders einfach, denn das Stützelement wird in seinem gestreckten Ausgangszustand zunächst außerhalb des Rohrschafts auf das Bauelement gebracht, oder die Bauelemente werden in dieses eingeschoben, und erst dann wird der Zusammenbau in den Rohrschaft eingeführt, wobei genügend radiales Spiel vorhanden ist, um diese Montage ohne weiteres zuzulassen, denn das schrumpffähige Material ist in diesem Zustand nur ein dünner Film. Erst durch die dann durchgeführte Wärmebehandlung nimmt das schrumpffähige Material in seiner Dicke zu und stellt den innigen Kontakt zwischen der Innenseite des Rohrschafts und der Außenseite des Bauelements her. Wenn Nuten, Stege, Erhebungen oder sonstiges auf einer der beteiligten Oberflächen vorgesehen sind, so werden diese von dem schrumpffähigen Material, das sich unter Wärmebehandlung in einem gummiartigen Zustand befindet, sozusagen "umschlossen" und "ausgefüllt".

In einer bevorzugten Fortbildung des erfindungsgemäßen Verfahrens werden mehrere Bauelemente axial hintereinander längs der Rohrschaftachse angeordnet, und ein sich über die mehreren Bauelemente hinweg erstreckendes Stützelement wird derart aufgebracht, daß die Bauelemente nach der Wärmebehandlung zusätzlich in der axialen Anordnung relativ zueinander fixiert werden.

Diese Maßnahme hat den Vorteil, ohne zusätzliche Maßnahmen die Fixierung der Bauelemente im Inneren des Rohrschafts sowie deren Verbinden und Fixierung relativ zueinander zu gewährleisten. Eine Aufreihung der Bauelemente außerhalb des Rohrschafts kann vorgenommen werden und dann mit dem Stützelement in Verbindung gebracht werden, was besonders einfach ist, da die Sicht nicht durch den Rohrschaft behindert wird. Erst anschließend wird der Zusammenbau in den Rohrschaft hineingeschoben.

Durch die Wärmebehandlung und die Materialeigenschaften des schrumpffähigen Materials wird eine gegen axiale und radiale Verschiebungen gesicherte Anordnung der Bauelemente innerhalb des Rohrschafts gewährleistet. So wird eine doppelte Fixierung der Bauelemente erreicht, nämlich die Fixierung an den Rohrschaft und die Fixierung relativ zueinander.

Weitere Vorteile und Ausgestaltungen der Erfindung werden nachstehend unter Bezugnahme auf die Figuren erläutert. Es zeigen:
- Fig. 1: eine teilweise aufgebrochene Seitenansicht eines erfindungsgemäßen Endoskops;
- Fig. 2: einen Ausschnitt des Endoskops von Fig. 1 im Längsschnitt in einer ersten Ausführungsform;
- Fig. 3: einen der Fig. 2 entsprechenden Ausschnitt eines erfindungsgemäßen Endoskops im Längsschnitt in einer zweiten Ausführungsform;
- Fig. 4: einen der Fig. 2 entsprechenden Ausschnitt eines erfindungsgemäßen Endoskops im Längsschnitt in einer dritten Ausführungsform;
- Fig. 5a: einen Querschnitt eines Stützelements in Ringform;
- Fig. 5b: einen Längsschnitt des Stützelements von Fig. 5a;
- Fig. 6a: einen Querschnitt eines Stützelements in Form eines Halbrings;
- Fig. 6b: einen Längsschnitt des Stützelements von Fig. 6a;
- Fig. 7a: einen Querschnitt von Stützelementen in Form von drei Längsstreifen;
- Fig. 7b: einen Längsschnitt der Anordnung von Fig. 7a;
- Fig. 8a: eine Frontansicht eines Stützelements in Form eines schraubenlinienförmig gewundenen Bandes;
- Fig. 8b: eine abschnittsweise Seitenansicht des Bandes von Fig. 8a;
- Fig. 9 bis 13: ausschnittsweise Längsschnitte von verschiedenen weiteren Ausführungsformen eines erfindungsgemäßen Endoskops;
- Fig. 14: einen ausschnittsweisen Längsschnitt einer weiteren Ausführungsform eines erfindungsgemäßen Endoskops; und
- Fig. 15: einen ausschnittsweisen Längsschnitt eines Endoskop des Standes der Technik.

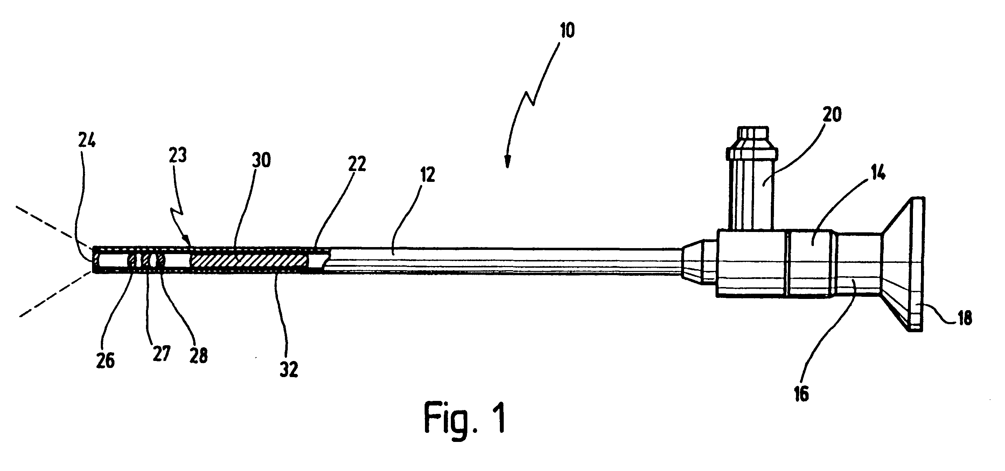

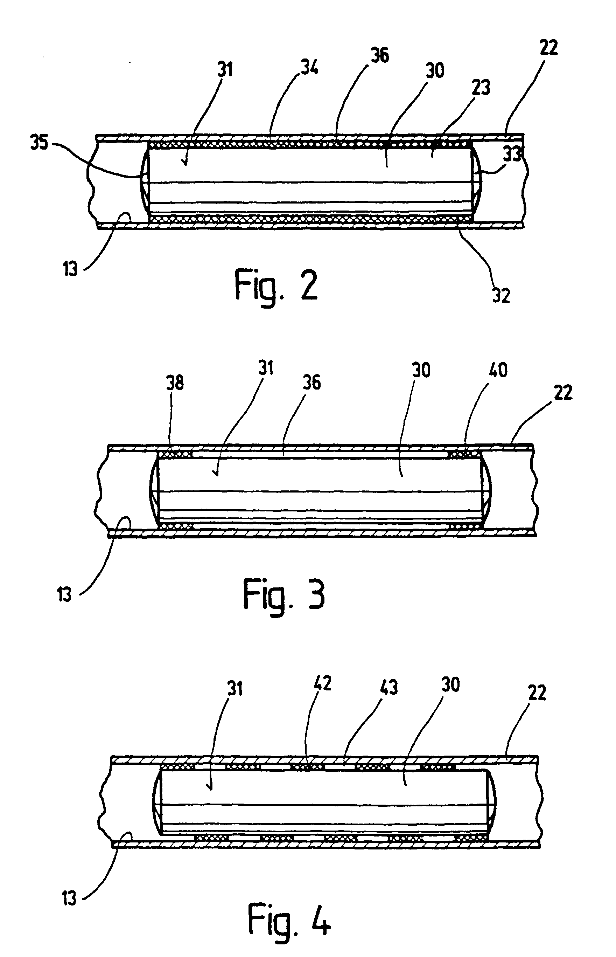

Ein in Fig. 1 und 2 dargestelltes Endoskop ist in seiner Gesamtheit mit der Bezugsziffer 10 bezeichnet.

Das Endoskop 10 weist einen langerstreckten rohrförmigen Schaft 12 auf, an dessen proximalem Ende ein Gehäuse 14 angeordnet ist. Im Gehäuse ist ein Okular 16 mit einer Okularmuschel 18 vorgesehen.

Im Endoskop 10 ist ein optisches System aufgenommen, das dazu dient, um ein am distalen Ende erfaßbares Bild zum proximalen Ende zu bringen. Im Rohrschaft 12 ist üblicherweise ein hier nicht näher bezeichnetes Lichtleitsystem in Form von Glasfasern angeordnet, die über einen seitlich vom Gehäuse 14 abführenden Ansatz 20 mit einer hier nicht dargestellten Lichtquelle verbunden sind. Im Rohrschaft 12 ist ein Innenrohr 22 angeordnet, das zahlreiche Bauelemente 23 des optischen Systems aufnimmt.

Distalseitig bildet ein Fenster 24 einen dichten Abschluß der distalen Seite des Innenrohrs 22, über das die Bildinformation in das optische System eindringt.

Im Innenrohr 22 sind zahlreiche Linsen 26, 27, 28, unter anderem auch stabförmige Linsen 30, wie das bspw. bei einer sogenannten HOPKINS-Optik der Fall ist, enthalten.

In Fig. 2 ist ein Abschnitt des Innenrohrs 22 dargestellt, in dem die stabförmige Linse 30 montiert ist. Die Stablinse 30 weist an ihren gegenüberliegenden Enden jeweils eine optische Fläche 33 bzw. 35 auf. Über ihre glatte Außenseite 31 ist ein Stützelement 32 in Form eines Schlauchabschnitts 34 geschoben.

Der Schlauchabschnitt 34 besteht aus einer Schlauchfolie aus kaltgerecktem PVC.

Bei der Montage wird zunächst der Schlauchabschnitt 34, der als relativ dünne Folie ausgebildet ist, über die Stablinse 30 geschoben. Anschließend wird der Zusammenbau aus Stablinse 30 und aufgeschobenem Schlauchabschnitt 34 in das Innenrohr 22 eingeschoben und in die in Fig. 2 dargestellte axiale Lage gebracht. Entsprechend kann dann mit den anderen Bauelementen des optischen Systems verfahren werden.

Anschließend wird der Zusammenbau aus Innenrohr, Stützelement 32 und Stablinse 30 einer solchen Wärmebehandlung unterworfen, bei der das schrumpffähige Material des Schlauchabschnitts 34 schrumpft. Bei diesem Schrumpfvorgang hat das Material die Tendenz, sich axial zusammenzuziehen und sich dabei radial etwas auszudehnen. Dadurch wird der Spalt 36 zwischen der Außenseite 31 der Stablinse 30 und der Innenseite 13 des Innenrohrs 22 vollständig mit dem erweichten schrumpfenden Material ausgefüllt. Dadurch entsteht ein Preßdruck, der radial zwischen Innenseite 13 des Innenrohrs 22 und Außenseite 31 der Stablinse 30 wirkt, so daß die Linse 30 axial unverrückbar im Innenrohr 22 fixiert ist. Nach Erkalten des Materials wird dies relativ steif, bleibt jedoch elastisch, so daß ein elastischer Mantel die Stablinse 30 umgibt. Durch diesen elastischen Mantel werden dann auch äußere Krafteinwirkungen abgefedert und dadurch die Bruchgefahr der Stablinse 30 erheblich herabgesetzt.

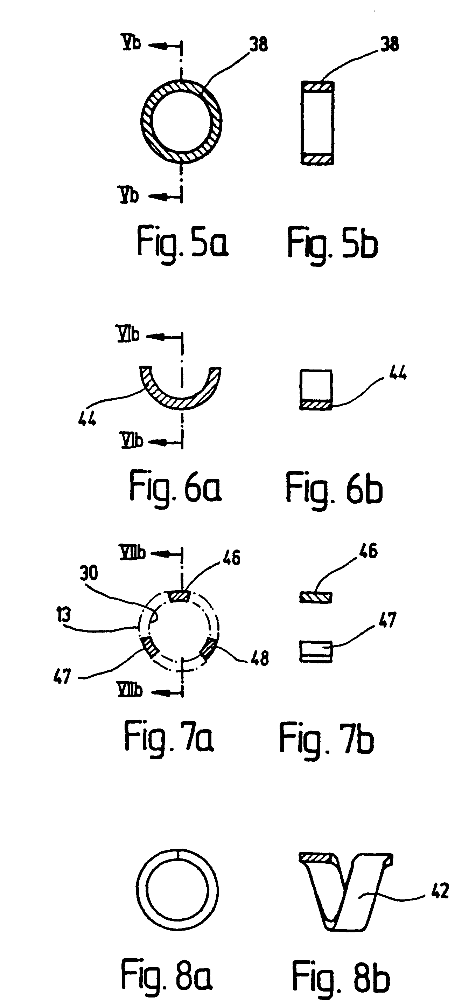

Aus Fig. 3 ist zu erkennen, daß es auch möglich ist, die Stablinse 30 über zwei an ihrem jeweiligen Endbereich angeordnete Ringe 38 und 40 zu montieren (siehe auch Fig. 5a, 5b).

Diese Zwei-Punktauflage erlaubt ebenfalls eine kippsichere und axial unverschiebliche Fixierung. In diesem Fall ist dann über einen langen axialen Bereich ein Luftspalt 36 zwischen der Außenseite 31 der Stablinse 30 und der Innenseite 13 des Innenrohrs 22 vorhanden.

Die Ringe 38 und 40 sowie die nachfolgend weiteren noch zu beschreibenden Stützelemente sind durchweg aus einem schrumpffähigen Material hergestellt, und die Montageweise ist gleich wie zuvor beschrieben.

Aus Fig. 4 in Zusammenhang mit den Fig. 8a und 8b ist zu erkennen, daß auch die Möglichkeit besteht, das Stützelement in Form eines schraubenlinienförmigen Bandes 42 um die Außenseite 31 der Stablinse 30 zu legen. Dadurch wird ein durchgehender schraubenlinienförmiger Spaltkanal 43 freigelassen, so daß ein Druckausgleich in den Bereichen beidseits der gegenüberliegenden Enden der Stablinse 30 möglich ist.

Aus Fig. 6a und 6b ist zu erkennen, daß ein Stützelement auch in Form eines Halbrings 44 oder, wie aus Fig. 7a und 7b zu erkennen ist, in Form von mehreren längsverlaufenden Streifen 46, 47 und 48 ausgebildet sein kann. Die beiden zuletzt genannten Konstruktionen erlauben ebenfalls eine ausreichende Abstützung und Fixierung und zugleich einen Druckausgleich oder auch den Durchtritt von Spülflüssigkeiten oder dgl.

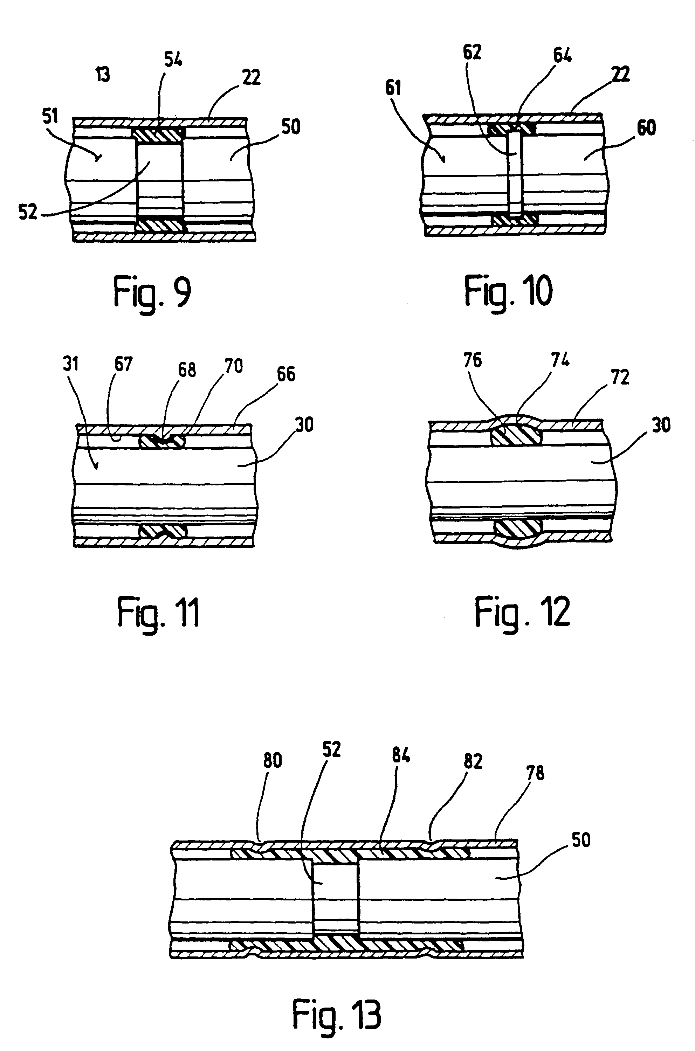

In den Fig. 9 bis 13 sind nun verschiedene Varianten der Ausgestaltung der Außenseite der Linsen bzw. der Innenseite des Innenrohrs dargestellt, um die eingangs erwähnte zusätzliche mechanische Verzahnung zu erzielen.

Aus Fig. 9 ist zu erkennen, daß die Stablinse 50 im Gegensatz zu der Stablinse 30 eine umfänglich in ihre Außenseite 51 eingeschnittene Nut 52 aufweist. Ein Stützelement in Form eines Rings 54 weist eine solche axiale Länge auf, daß es sich beidseits über die Nut 52 hinaus erstreckt. Die Innenseite 13 des Innenrohrs 22 ist glatt. In dieser Ausgestaltung ist die Montage sehr einfach, denn der Ring 54 muß lediglich auf die Linse 50 geschoben werden, bis er an dessen Nut 52 gelangt. Nach Einschieben des Zusammenbaus und Durchführen der Wärmebehandlung schrumpft der Ring 54 und preßt sich besonders fest in den Ringraum zwischen Nut 52 und Innenseite 13 des Innenrohrs 22 hinein. Dadurch entsteht eine mechanische Verzahnung, die eine axiale Relativbewegung zwischen Ring 54 und Linse 50 sperrt.

In Fig. 10 wird ein solches Verzahnen dadurch erreicht, daß an einer Außenseite 61 einer Stablinse 60 ein umlaufender Steg 62 vorgesehen ist. Auch hier ist die Innenseite 13 des Innenrohrs 22 glatt. Auch hier entsteht nach der Wärmebehandlung eine intensive mechanische Verzahnung zwischen einem Ring 64 und dem Steg 62, die eine axiale relative Verschiebung dieser beiden Bauelemente zueinander sperrt.

Aus Fig. 11 ist zu erkennen, daß an der Innenseite 67 des Innenrohrs 66 ein radial nach innen vorspringender Ringflansch 68 vorgesehen ist. Ein um die Außenseite 31 der glatten Stablinse 30 gelegter Ring 70 geht eine innige mechanische Verzahnung mit dem Ringflansch 68 nach der Wärmebehandlung ein. Dadurch wird eine Relativverschiebung in axialer Richtung zwischen Ring 70 und Innenrohr 66 mechanisch gesperrt.

Eine ähnliche mechanische Sperrung wird auch mit der Ausgestaltung von Fig. 12 erzielt. In diesem Fall ist im Innenrohr 72 eine nach außen gerichtete Ausbauchung 74 vorgesehen, in die sich ein Ring 76 nach der Wärmebehandlung formschlüssig einschmiegt. In diesem Fall kann ebenfalls die Stablinse 30 mit der glatten Außenseite montiert werden.

In Fig. 13 ist ein Innenrohr 78 dargestellt, in dem zwei axial voneinander beabstandete Sicken 80 und 82 vorgesehen sind. Im Innenrohr 78 ist die bereits in Zusammenhang mit Fig. 9 beschriebene Stablinse 50 mit der umfänglich eingeschnittenen Nut 52 montiert.

Dazu ist über die Stablinse 50 ein Schlauchstück 84 aus dem zuvor beschriebenen schrumpffähigen Material geschoben, dessen axiale Länge so gewählt ist, daß er sowohl die Nut 52 als auch die Sicken 80 und 82 axial überragt.

Nach der Wärmebehandlung besteht eine intensive mechanische Verzahnung zwischen dem Schlauchstück 84 und den Sicken 80 und 82, wodurch eine Relativverschiebung in axialer Richtung zwischen Innenrohr 78 und Schlauchstück 84 gesperrt ist. Gleichzeitig hat sich eine innige mechanische Verzahnung zwischen dem Schlauchstück 84 und der Nut 52 ausgebildet, wodurch eine Relativverschiebung zwischen Schlauchstück 84 und Linse 50 gesperrt ist. Dadurch ist der Zusammenbau bzw. die Linse 50 besonders sicher in axialer Lage fixiert. Diese mehreren Verzahnungsstellen ermöglichen auch, Endoskope mit relativ großem Durchmesser mit relativ dünnen Schlauchstücken 84 bzw. relativ dünnen Folien zu fixieren.

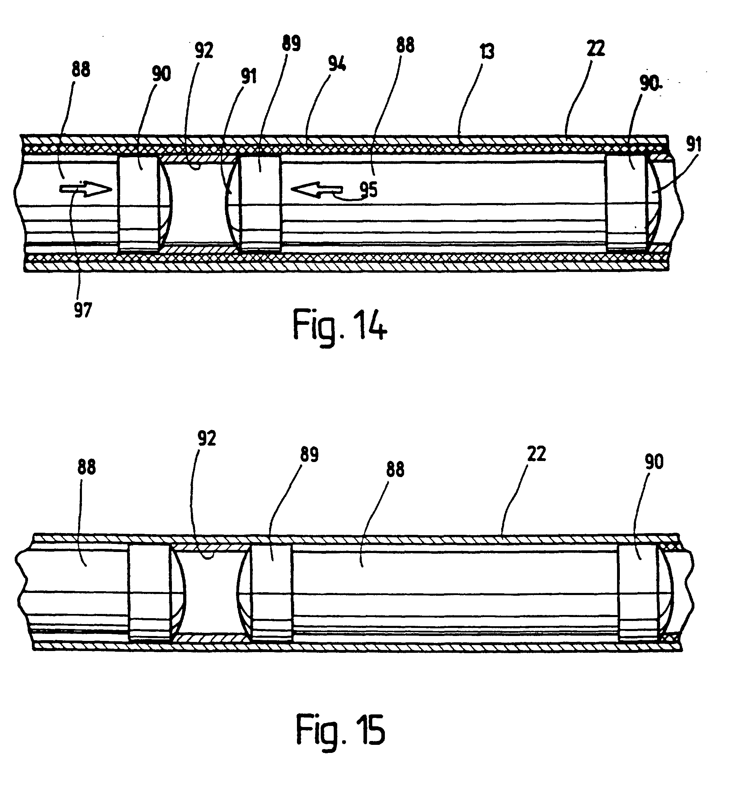

In Fig. 14 ist dargestellt, wie mehrere Knochenlinsen 88 hintereinanderliegend entsprechend der vorliegenden Erfindung montiert werden können.

Jede Knochenlinse 88 weist einen Randzylinder 89 bzw. 90 auf.

Endseitig sind die optischen Flächen 91 vorgesehen. Zwischen den beiden Knochenlinsen 88 ist ein Distanzstück 92 in Form eines Rohrstückes gelegt, das für eine axiale Beabstandung sorgt. Über den Zusammenbau aus Knochenlinsen 88 und Distanzstück 92 ist ein Stützelement entsprechend der vorliegenden Erfindung in Form eines Schlauches 94 geschoben bzw. diese Elemente wurden nach und nach in den Schlauch eingeschoben.

Nachdem dieser Zusammenbau in bspw. das Innenrohr 22 eingeschoben wurde, bewirkt die Schrumpfkraft den weiteren Vorteil, daß die beiden Knochenlinsen 88 etwas aufeinander zu gedrückt werden, wie das durch Pfeile 95 und 97 angedeutet ist, somit auch die Relativlage unter einem zusätzlichen Anpreßdruck fixiert wird. Aus Fig. 14 ist zu erkennen, daß die Randzylinder 89 und 90 sich nunmehr auf dem Schlauch 94 abstützen und nicht mehr direkt auf der Innenseite 13 des Innenrohrs 22. Bei der Montage solcher Knochenlinsen 88, wie das nach dem Stand der Technik bekannt war, wie das aus Fig. 15 ersichtlich ist, treten die Randzylinder 89 und 90 direkt mit der Innenseite des Innenrohrs 22 in Berührung, so daß möglicherweise von außen einwirkende Kräfte direkt auf die doch brüchigen Linsen aus Glasmaterialien treffen können.

Damit die Knochenlinsen 88 in dem gewünschten Abstand der axialen Länge des Distanzstückes 92 zueinander zum Liegen kommen, ist es dann notwendig, am proximalen und distalen Ende des Innenrohrs, bei der Montage entsprechend dem Stand der Technik, einen entsprechenden Anpreßdruck aufzubringen.

Dies wird nunmehr zusätzlich durch die Schrumpfbewegung der Folie unterstützt.

## Patentansprüche

1. Endoskop (10) mit einem Rohrschaft (22), in dessen Innerem zumindest ein Bauelement (23), insbesondere Linsen (26, 27, 28, 30; 50; 60; 88), Blenden, Filter, eines optischen Systems angeordnet ist, wobei auf der Außenseite (31, 51, 61) des Bauelements (23) zumindest ein das Bauelement (23) zumindest teilweise umgebendes Stützelement (32) aus schrumpffähigem Material vorhanden ist, das einen radialen Spalt (36) zwischen der Außenseite (31, 51, 61) des Bauelementes (23) und der Innenseite (13) des Rohrschaftes (22) ausfüllt, **dadurch gekennzeichnet, daß** der radiale Spalt (36) nach dem Schrumpfen des Stützelementes (32) so ausgefüllt ist, daß das Bauelement über das geschrumpfte Stützelement (32) an der Innenseite (13) des Rohrschaftes (22) fixiert ist.

2. Endoskop nach Anspruch 1, **dadurch gekennzeichnet, daß** das Stützelement aus einem Schlauchabschnitt (34, 84) besteht, der einen Großteil der Außenseite (31) des Bauelements (23) bedeckt.

3. Endoskop nach Anspruch 1, **dadurch gekennzeichnet, daß** das Stützelement aus zumindest einem Ring (38, 40; 54, 64, 70, 76) besteht.

4. Endoskop nach Anspruch 3, **dadurch gekennzeichnet, daß** das Stützelement aus zwei Ringen (38, 40) besteht, die an gegenüberliegenden Enden des Bauteils angeordnet sind.

5. Endoskop nach Anspruch 1, **dadurch gekennzeichnet, daß** das Stützelement aus zumindest einem Halbring (44) besteht.

6. Endoskop nach Anspruch 1, **dadurch gekennzeichnet, daß** das Stützelement aus zumindest zwei Streifen (46, 47, 48) besteht, die sich in Längsrichtung des Rohrschafts (22) erstrecken.

7. Endoskop nach Anspruch 1, **dadurch gekennzeichnet, daß** das Stützelement aus einem schraubenlinienförmig gewundenen Band (42) besteht.

8. Endoskop nach einem der Anspüche 1 bis 7, **dadurch gekennzeichnet, daß** die Außenseite (31) des Bauelements glatt ist.

9. Endoskop nach einem der Ansprüche 1 bis 8, **dadurch gekennzeichnet, daß** in die Außenseite des Bauelements zumindest eine umfängliche Nut (52) eingeschnitten ist und das Stützelement im Bereich der Nut (52) angeordnet ist.

10. Endoskop nach einem der Ansprüche 1 bis 8, **dadurch gekennzeichnet, daß** an der Außenseite des Bauelements zumindest ein Steg (62) vorgesehen ist und daß das Stützelement im Bereich des Stegs (62) angeordnet ist.

11. Endoskop nach einem der Ansprüche 1 bis 10, **dadurch gekennzeichnet, daß** die Innenseite (13) des Rohrschafts (22) glatt ist.

12. Endoskop nach einem der Ansprüche 1 bis 10, **dadurch gekennzeichnet, daß** die Innenseite des Rohrschafts zumindest eine Nut aufweist und das Stützelement im Bereich der Nut angeordnet ist.

13. Endoskop nach einem der Ansprüche 1 bis 10, **dadurch gekennzeichnet, daß** die Innenseite des Rohrschafts (72) zumindest eine nach außen gerichtete Ausbauchung (74) in Form eines Kugelabschnitts aufweist und das Stützelement im Bereich der Ausbauchung (74) angeordnet ist.

14. Endoskop nach einem der Ansprüche 1 bis 10, **dadurch gekennzeichnet, daß** an der Innenseite (67) des Rohrschafts (66) zumindest ein Ringflansch (68) vorgesehen ist und das Stützelement im Bereich des Ringflansches (68) angeordnet ist.

15. Endoskop nach einem der Ansprüche 1 bis 14, **dadurch gekennzeichnet, daß** mehrere Bauelemente axial hintereinanderliegend im Inneren des Rohrschafts (22) längs der Rohrschaftachse angeordnet sind, daß das Stützelement sich über die mehreren Bauelemente hinweg erstreckt und daß diese durch das geschrumpfte Stützelement in einer axialen Anordnung untereinander fixiert sind.

16. Verfahren zum Montieren von Bauelementen, insbesondere von Linsen (26, 27, 28, 30; 50; 60; 88), Blenden, Filtern eines optischen Systems im Inneren eines Rohrschafts (22) eines Endoskops (10), bei dem auf die Außenseite (31) des Bauelements (23) zumindest ein das Bauelement (32) zumindest teilweise umgebendes Stützelement (32) aus schrumpffähigem Material gebracht wird und dieser Zusammenbau einer Wärmebehandlung unterzogen wird, durch die das Stützelement (32) schrumpft und so das Bauelement (23) fixiert, und bei dem nach dem Einführen des Zusammenbaus in den Rohrschaft (22) der radiale Spalt (36) zwischen der Außenseite (31, 51, 61) des Bauelementes (23) und der Innenseite (13) des Rohrschaftes (22) von dem Stützelement (32) ausgefüllt ist, **dadurch gekennzeichnet, daß** der Zusammenbau vor der Wärmebehandlung in den Rohrschaft (22) eingeführt wird, daß zwischen der Außenseite (31, 51, 61) des Bauelementes (23) und der Innenseite (13) des Rohrschaftes (22) vor der Wärmebehandlung ein radialer Spalt (36) freigelassen wird, der nach dem Schrumpfen des Stützelementes (32) so ausgefüllt ist, daß das Bauelement über das geschrumpfte Stützelement (32) an der Innenseite (13) des Rohrschaftes (22) fixiert wird.

17. Verfahren nach Anspruch 16, **dadurch gekennzeichnet, daß** mehrere Bauelemente axial hintereinander längs der Rohrschaftachse angeordnet werden und ein sich über die mehreren Bauelemente hinweg erstreckendes Stützelement (92) derart aufgebracht wird, daß die Bauelemente nach der Wärmebehandlung zusätzlich in der axialen Anordung relativ untereinander fixiert sind.

## Claims

1. Endoscope (10) having a tubular shaft (22) in whose interior is arranged at least one component (23), in particular lenses (26, 27, 28, 30; 50; 60; 88), diaphragms, filters of an optical system, at least one support element (32) made of a shrinkable material is provided at the outer side (31, 51, 61) of the component (23) which at least partially surrounds the component (23) and which support element (32) fills a radial gap (36) between the outer side (31, 51, 61) of the component (23) and the inner side (13) of the tubular shaft (22), **characterized in that** the radial gap (36) is filled up after a shrinking of the support element (32) in such a manner that the component is fixed to the inner side (13) of the tubular shaft (22) via the shrunken support element (32).

2. Endoscope of Claim 1, **characterized in that** the support element is a hose section (34, 84) covering a majority of the outer side (31) of the component (23).

3. Endoscope of Claim 1, **characterized in that** the support element is designed of at least one ring (38, 40; 54, 64, 70, 76).

4. Endoscope of Claim 3, **characterized in that** the support element is designed of two rings (38, 40) that are arranged at opposite ends of the component.

5. Endoscope of Claim 1, **characterized in that** the support element is designed as at least one half-ring (44).

6. Endoscope of Claim 1, **characterized in that** the support element is designed of at least two strips (46, 47, 48) that extend in the longitudinal direction of the tubular shaft (22).

7. Endoscope of Claim 1, **characterized in that** the support element is designed of a band (42) wound in helical fashion.

8. Endoscope of anyone of Claims 1 through 7, **characterized in that** the outer side (31) of the component is smooth.

9. Endoscope of anyone of Claims 1 through 8, **characterized in that** at least one circumferential groove (52) is cut into the outer side of the component, and **in that** the support element is arranged in the region of the groove (52).

10. Endoscope of anyone of Claims 1 through 8, **characterized in that** at least one land (62) is provided on the outer side of the component, and **in that** the support element is arranged in the region of the land (62).

11. Endoscope of anyone of Claims 1 through 10, **characterized in that** the inner side (13) of the tubular shaft (22) is smooth.

12. Endoscope of anyone of Claims 1 through 10, **characterized in that** the inner side of the tubular shaft has at least one groove, and **in that** the support element is arranged in the region of the groove.

13. Endoscope of anyone of Claims 1 through 10, **characterized in that** the inner side of the tubular shaft (72) has at least one outwardly directed protrusion (74) in the form of a spherical segment, and **in that** the support element is arranged in the region of the protrusion (74).

14. Endoscope of anyone of Claims 1 through 10, **characterized in that** at least one annular flange (68) is provided on the inner side (67) of the tubular shaft (66), and **in that** the support element is arranged in the region of the annular flange (68).

15. Endoscope of anyone of Claims 1 through 14, **characterized in that** several components are arranged axially one behind another in the interior of the tubular shaft (22) along the tubular shaft axis; the support element extends over the several components; and **in that** the latter are immobilized with respect to one another in an axial arrangement by the shrunken support element.

16. Method for mounting components, in particular lenses (26, 27, 28, 30; 50; 60; 88), diaphragms, filters of an optical system in the interior of a tubular shaft (22) of an endoscope (10), in which at least one support element (32) made of shrinkable material that at least partially surrounds the component (23) is disposed on the outer side (31) of the component (23) and wherein this assembly is burden to a heat treatment, due to which the support element (32) shrinks and fixes the component (23), and wherein after inserting the assembly into the tubular shaft, a radial gap (36) between the outer side (31, 51, 61) of the component (23) and the inner side (13) of the tubular shaft (32) is filled by the support element (32), **characterized in that** the assembly is inserted into the tubular shaft (22) prior to the heat treatment and **in that** prior to the heat treatment, a radial gap (36) is set free between the outer side (31, 51, 61) of the component (23) and the inner side (13) of the tubular shaft (22), which gap is filled after shrinking the support element (32) in such a manner that the component is fixed at the inner side (13) of the tubular shaft (22) via the shrunken support element (32).

17. Method of Claim 16, **characterized in that** several components are arranged axially one behind another along the tubular shaft axis, and a support element (92) extending over the several components is applied in such a way that after the heat treatment, the components are additionally immobilized relative to one another in their axial arrangement.

## Revendications

1. Endoscope (10) avec une tige tubulaire (22) à l'intérieur de laquelle est au moins disposé un composant (23), notamment des lentilles (26, 27, 28, 30 ; 50 ; 60 ; 88), des diaphragmes, des filtres, d'un système optique, sur la face externe (31, 51, 61) du composant (23) se trouvant au moins un support (32), entourant au moins partiellement le composant (23), fabriqué dans un matériau rétractile, élément qui remplit une fente radiale (36) entre la face externe (31, 51, 61) du composant (23) et la face interne (13) de la tige tubulaire (22), **caractérisé en ce que** la fente radiale (36) est remplie, après la rétraction du support (32), de telle sorte que le composant soit fixé à la face interne (13) de la tige tubulaire (22) par le biais du support (32).

2. Endoscope selon la revendication 1, **caractérisé en ce que** le support est composé d'un tronçon de tuyau (34, 84) qui recouvre une grande partie de la face externe (31) du composant (23).

3. Endoscope selon la revendication 1, **caractérisé en ce que** le support est composé d'au moins une bague (38, 40 ; 54, 64, 70, 76).

4. Endoscope selon la revendication 3, **caractérisé en ce que** le support est composé de deux bagues (38, 40) qui sont disposées à des extrémités opposées du composant.

5. Endoscope selon la revendication 1, **caractérisé en ce que** le support est composé d'au moins une demi-bague (44).

6. Endoscope selon la revendication 1, **caractérisé en ce que** le support est composé d'au moins deux bandes (46, 47, 48) qui s'étendent dans le sens longitudinal de la tige tubulaire (22).

7. Endoscope selon la revendication 1, **caractérisé en ce que** le support est composé d'une bande en hélice (42).

8. Endoscope selon l'une des revendications 1 à 7, **caractérisé en ce que** la face externe (31) du composant est lisse.

9. Endoscope selon l'une des revendications 1 à 8, **caractérisé en ce que** dans la face externe du composant est creusée au moins une rainure circonférentielle (52) et que le support est disposé au niveau de la rainure (52).

10. Endoscope selon l'une des revendications 1 à 8, **caractérisé en ce que** sur la face externe du composant est prévue au moins une nervure (62) et que le support est disposé au niveau de la nervure (62).

11. Endoscope selon l'une des revendications 1 à 10, **caractérisé en ce que** la face interne (13) de la tige tubulaire (22) est lisse.

12. Endoscope selon l'une des revendications 1 à 10, **caractérisé en ce que** la face interne de la tige tubulaire comprend au moins une rainure et que le support est disposé au niveau de la rainure.

13. Endoscope selon l'une des revendications 1 à 10, **caractérisé en ce que** la face interne de la tige tubulaire (72) comprend au moins un bossage (74) tourné vers l'extérieur, sous la forme d'un segment sphérique, et que le support est disposé au niveau du bossage (74).

14. Endoscope selon l'une des revendications 1 à 10, **caractérisé en ce que** sur la face interne (67) de la tige tubulaire (66) est prévue une bride annulaire (68) et que le support est disposé au niveau de la bride annulaire (68).

15. Endoscope selon l'une des revendications 1 à 14, **caractérisé en ce que** plusieurs composants sont disposés les uns derrière les autres, dans une direction axiale, à l'intérieur de la tige tubulaire (22), le long de l'axe de la tige tubulaire, que le support s'étend sur les différents composants et que ces derniers sont fixés les uns aux autres, dans une disposition axiale, par le support rétracté.

16. Procédé pour le montage de composants, notamment de lentilles (26, 27, 28, 30 ; 50 ; 60 ; 88), diaphragmes et filtres d'un système optique à l'intérieur d'une tige tubulaire (22) d'un endoscope (10), dans lequel, sur la face externe (31) du composant (23) est installé au moins un support (32), entourant le composant (23) au moins partiellement, fabriqué dans un matériau rétractile et cet assemblage est soumis à un traitement thermique par lequel le support (32) se rétracte et fixe ainsi le composant (23), et dans lequel, après l'introduction de l'assemblage dans la tige tubulaire (22), la fente radiale (36), entre la face externe (31, 51, 61) du composant (23) et la face interne (13) de la tige tubulaire (22), est remplie par le support (32), **caractérisé en ce que** l'assemblage est introduit avant le traitement thermique dans la tige tubulaire (22), **en ce qu'**entre la face externe (31, 51, 61) du composant (23) et la face interne (13) de la tige tubulaire (22), une fente radiale (36) est laissée libre avant le traitement thermique, fente qui est remplie après la rétraction du support (32) de telle sorte que le composant soit fixé à la face interne (13) de la tige tubulaire (22) par le biais du support rétracté (32).

17. Procédé selon revendication 16, **caractérisé en ce que** plusieurs composants sont disposés les uns derrière les autres, dans une direction axiale, le long de l'axe de la tige tubulaire et qu'un support (92) s'étendant sur les différents composants est placé de telle sorte que les composants soient fixés en outre dans la disposition axiale, les uns par rapport aux autres, après le traitement thermique.
